Europäisches Patentamt

European Patent Office

Office européen des brevets

(11) Veröffentlichungsnummer : **0 030 627**
**B1**

(19)

(12) **EUROPÄISCHE PATENTSCHRIFT**

(45) Veröffentlichungstag der Patentschrift :
15.12.82

(51) Int. Cl.³ : **C 07 D249/12**

(21) Anmeldenummer : **80107047.5**

(22) Anmeldetag : **14.11.80**

(54) **Verfahren zur Herstellung von 1,2,4-Triazolidin-3,5-dion.**

(30) Priorität : 26.11.79 DE 2947619

(43) Veröffentlichungstag der Anmeldung :
24.06.81 (Patentblatt 81/25)

(45) Bekanntmachung des Hinweises auf die Patenterteilung : 15.12.82 Patentblatt 82/50

(84) Benannte Vertragsstaaten :
CH DE FR GB IT LI

(56) Entgegenhaltungen :
FR A 1 447 532
CHEMICAL ABSTRACTS, Band 67, nr. 25, 18.
Dezember 1967, Seite 11 009, Zusammenfassung
Nr. 116858y, Columbus, Ohio, US, M. FURDIK et
al. : « Synthesis of azodicarboxylic acid imide, its
N-substituted derivatives and their use as dienophiles in the Diels-Alder reaction ».
CANADIAN JOURNAL OF CHEMISTRY, Band 50,
Nr. 11, 1972, J. LENOIR et al. : « Hydrazine derivatives. I. Transcarbamylation reaction », Seiten
2 661-2 664.

(73) Patentinhaber : **BAYER AG**
**Zentralbereich Patente, Marken und Lizenzen**
**D-5090 Leverkusen 1, Bayerwerk (DE)**

(72) Erfinder : **Merten, Rudolf, Dr.**
**Berta-von-Suttner-Strasse 55**
**D-5090 Leverkusen 1 (DE)**
Erfinder : **Rottmaier, Ludwig, Ing.-grad.**
**Bergstrasse 85**
**D-5068 Odenthal (DE)**

Verfahren zur Herstellung von 1,2,4-Triazolidin-3,5-dion

Die vorliegende Erfindung betrifft ein Verfahren zur Herstellung von 1,2,4-Triazolidin-3,5-dion durch Erhitzen von Hydrazodicarbonamid in mindestens einem organischen Lösungsmittel.

Die Herstellung von 1,2,4-Triazolidin-3,5-dion aus Hydrazodicarbonamid ist bekannt. Sie wird (wie in Liebigs Annalen der Chemie Bd. 283, s. 41 (1894) beschrieben) durch Erhitzen von Hydrazodicarbonamid in der Schmelze durchgeführt. Diese Methode ist im technischen Maßstab jedoch kaum durchführbar. Die erhaltene Schmelze ist stark verunreinigt und fällt nach dem Erkalten als feste, harte Masse an, die zur weiteren Verarbeitung zerkleinert und gereinigt werden muß. Die Ausbeuten an 1,2,4-Triazolidin-3,5-dion liegen bei 40 bis 50 % der Theorie.

Aufgabe der Erfindung war es, ein Verfahren zur Herstellung von 1,2,4-Triazolidin-3,5-dion aus Hydrazodicarbonamid aufzufinden, welches technisch leicht durchführbar ist, und durch welches 1,2,4-Triazolidin-3,5-dion in kristalliner Form von hoher Reinheit und Ausbeute erhältlich ist.

Die Aufgabe wurde dadurch gelöst, daß Hydrozodicarbonamid in organischen Lösungsmitteln (= organischen Reaktionsmedien) unter speziellen Verfahrensbedingungen cyclisiert wurde.

Gegenstand der vorliegenden Erfindung ist somit ein Verfahren zur Herstellung von 1,2,4-Triazolidin-3,5-dion aus Hydrazodicarbonamid durch Cyclisierung bei erhöhten Temperaturen unter Abspaltung von Ammoniak, dadurch gekennzeichnet, daß Hydrazodicarbonamid in mindestens einem organischen, gegebenenfalls mit Wasser mischbaren Lösungsmittel suspendiert und bei Temperaturen von 150 °C bis 280 °C und einem Druck von 50 mbar bis 5 bar unter Entfernung des abgespaltenen Ammoniaks aus dem Reaktionsgemisch cyclisiert und das entstandene 1,2,4-Triazolidin-3,5-dion nach Kristallisation isoliert wird.

Das erfindungsgemäße Verfahren führt in einheitlicher Weise und in hohen Ausbeuten zum 1,2,4-Triazolidin-3,5-dion ohne Bildung von (polymeren) Nebenprodukten. Das Lösungsmittelmedium kann über zahlreiche Reaktionscyclen meistens ohne Reinigung wieder verwendet werden. Ein weiterer bzw. spezifischer Vorteil besteht in der Verwendung von feuchtem, rohem Ausgangsmaterial, das ohne Trocknungsprozess eingesetzt werden kann.

Die beim erfindungsgemäßen Verfahren zu verwendenden organischen Lösungsmittel sollen unter Reaktionsbedingungen eine ausreichende thermische Stabilität aufweisen und chemisch inert gegen Hydrazodicarbonamid bzw. Triazolidin-3,5-dion sein, auch soll der Siedepunkt genügend hoch liegen, damit das Lösungsmittel während der Reaktion nicht abdestilliert. Die Siedepunkte der Lösungsmittel liegen in der Regel bei Atmosphärendruck bei mindestens 150 °C, vorzugsweise bei ca. 200 °C bis 250 °C. Die Lösungsmittel können bei Raumtemperatur mit Wasser mischbar, zum Teil mischbar oder nicht mischbar sein.

Da das Hydrazodicarbonamid in den Lösungsmitteln praktisch nicht löslich ist und auch das Cyclisierungsprodukt 1,2,4-Triazolidin-3,5-dion in den Lösungsmitteln manchmal nur eine begrenzte Löslichkeit besitzt, so daß das Reaktionsgemisch nach der Cyclisierung vor der Kristallisation eine Emulsion darstellen kann, kann das Lösungsmittel auch als Reaktionsmedium bezeichnet werden.

Geeignete Lösungsmittel sind

A. mit Phenyl oder $C_1$-$C_6$-Alkylgruppen, N-substituierte stickstoffhaltige Lösungsmittel, zum Beispiel N-substituierte Pyrrolidone, Urethane, cyclische Urethane oder Harnstoffe, etwa N-Methylpyrrolidon, Ethyl-phenyl-urethan, 5-Methyl-2-oxazolidinon, Tetramethylharnstoff ; weiter Polyether, z.B. Diethylenglykoldiethylether ; Phenole wie Kresole, halogensubstituierte Phenole und Kresole, z.B. 4-Chlorphenol ; Dialkylsulfone und cyclische Sulfone jeweils mit maximal 12 Kohlenstoffatomen, z.B. Dimethylsulfon oder Sulfolan ; aromatische oder araliphatische Ether wie Diphenylether und Dibenzylether.

Geeignete Lösungsmittel sind ferner

B. aliphatische, cycloaliphatische, aromatische und araliphatische Kohlenwasserstoffe sowie deren technische Gemische wie z.B. Dodecan, Decalin, Trimethylbenzol, Naphthalin, 1-Methyl-naphthalin, Diphenylmethan, halogenierte aliphatische, cycloaliphatische, aromatische und araliphatische Kohlenwasserstoffe sowie deren technische Gemische wie Dodecylchlorid, 1,2,4-Trichlorbenzol, 1-Chlornaphthalin, Dichlor-toluol.

Besonders bevorzugt sind Diphenylether, Diphenylmethan, 1-Methylnaphthalin, Dialkylsulfone und cyclische Sulfone, insbesondere Sulfolan.

Die mit den polaren Lösungsmitteln, wie vorstehend unter A. aufgeführt, nach der Cyclisierung erhaltenen Reaktionsmischungen können während des Abkühlungsvorgangs mit gegen 1,2,4-Triazolidin-3,5-dion inerten Lösungsmitteln wie aliphatischen, cycloaliphatischen, araliphatischen oder aromatischen Kohlenwasserstoffen, z.B. Cyclohexan, Toluol, Xylol, aliphatischen oder cycloaliphatischen Alkoholen, z.B. Butanol, Cyclohexanol und daraus abgeleiteten Ethern oder Estern, z.B. Glykolmonomethylether, Essigsäurebutylester, Ketone z.B. Aceton oder Ethylmethylketon und bei Verwendung von mit Wasser mischbaren polaren Lösungsmitteln auch Wasser, abgemischt werden, so daß das auskristallisierende 1,2,4-Triazolidin-3,5-dion in größeren Ausbeuten bzw. höheren Reinheiten anfällt. Die Zusätze betragen bis zu 500 Gew.-%, bezogen auf polares Lösungsmittel.

Das bei dem erfindungsgemäßen Verfahren einzusetzende Hydrazodicarbonamid ist aus der Literatur bekannt und wird bei der Reaktion von 1 Mol Hydrazin mit 2 Mol Harnstoff unter Ammoniakabspaltung in wäßrigem Medium in praktisch quantitativer Ausbeute erhalten. Das dabei als Niederschlag anfallende Hydrazodicarbonamid wird durch Absaugen isoliert und kann als nutschenfeuchte Ware sofort weiterverarbeitet werden, wenn das Restwasser beim Cyclisierungsprozeß abgeführt werden kann. Selbstverständlich kann auch getrocknetes Hydrazodicarbonamid zur weiteren Reaktion verwendet werden. Es ist auch möglich, aus der erhaltenen Suspension aus Hydrazodicarbonamid in Wasser nach Zusatz eines geeigneten, erfindungsgemäßen Lösungsmittels durch Erhitzen das Wasser abzudestillieren und das verbleibende Hydrazodicarbonamid zum 1,2,4-Triazolidin-3,5-dion zu cyclisieren.

Für die Cyclisierung wird das Hydrazodicarbonamid in mindestens einem der angegebenen organischen Lösungsmittel suspendiert. Die Einsatz-Konzentrationen betragen vorzugsweise 10 bis 70 Gew.-%, d.h., in 100 g Reaktionsmischung sind 10 bis 70 g Hydrazodicarbonamid enthalten. Besonders bevorzugt sind Einsatzkonzentrationen des Hydrazodicarbonamids von 10 bis 50 Gew.-%.

Beim erfindungsgemäßen Verfahren liegt die Reaktions-temperatur im allgemeinen zwischen 150 und 280 °C, vorzugsweise zwischen 170 und 250 °C und insbesondere zwischen 190 und 220 °C. Je höher die Temperatur ist, desto schneller verläuft die Reaktion, allerdings nimmt die Gefahr der Bildung unerwünschter Nebenprodukte sowie die Zersetzung des Lösungsmittels zu.

Die Reaktionszeiten liegen im allgemeinen zwischen 0,5 und 10 Stunden, können jedoch in Ausnahmefällen auch darüber oder darunter liegen.

Der Reaktionsdruck beim erfindungsgemäßen Verfahren liegt normalerweise bei 50 mbar bis 5 bar, wobei bei höherem Druck als bei atmosphärischem Druck zwischendurch das entstehende Ammoniak abgelassen werden muß, so daß die Cyclisierung vorzugsweise bei 150 mbar bis atmosphärischem Druck durchgeführt wird.

Beim erfindungsgemäßen Verfahren ist es von Vorteil, wenn die Konzentration an abgespaltenem Ammoniak im Reaktionsgefäß niedrig gehalten wird. Dies kann auf jede bekannte Weise erfolgen, z.B. durch Ausblasen mit einem inerten Gas wie Luft, Stickstoff, Kohlendioxid oder Wasserdampf. Auch können niedrigsiedende Lösungsmittel, z.B. aliphatische, aromatische, araliphatische Kohlenwasserstoffe, deren technische Mischungen und chlorierte Kohlenwasserstoffe mit vorzugsweise 1-10 Kohlenstoffatomen wie Cyclohexan, Toluol, Xylole, Petrolether oder Chloroform, die flüssig in den Reaktor gepumpt oder getropft werden, zum Austreiben des Ammoniaks verwendet werden. Der Partialdruck des Ammoniaks kann auch durch Absaugen herabgesetzt werden, indem man bei unteratmosphärischem Druck arbeitet.

Das erfindungsgemäße Verfahren ist sowohl für diskontinuierliche als auch für kontinuierliche Arbeitsweise geeignet. Bei der kontinuierlichen Arbeitsweise wird die Cyclisierung nach bekannten Verfahren, z.B. durch die Benutzung von Kaskaden oder der Verwendung von Röhrenreaktoren, durchgeführt. Das diskontinuierliche Verfahren ist bevorzugt.

Wird das Verfahren in mit Wasser mischbaren, polaren Lösungsmitteln durchgeführt, so erfolgt die Aufarbeitung des 1,2,4-Triazolidin-3,5-dions meistens so, daß aus der erhaltenen heißen Triazolidin-3,5-dion-Lösung beim Abkühlen das 1,2,4-Triazolidin-3,5-dion auskristallisiert, welches durch Absaugen in praktisch reiner Form erhalten wird. In der Regel kann dieses technische 1,2,4-Triazolidin-3,5-dion sofort weiterverarbeitet werden, es kann aber, sofern ein besonders reines Produkt gewünscht wird, z.B. aus Wasser umkristallisiert werden.

Eine besonders bevorzugte Ausführungsform ist die Herstellung von 1,2,4-Triazolidin-3,5-dion aus Hydrazodicarbonamid in Sulfolan bei Temperaturen von 200-210 °C und einem durch Wasserstrahlvakuum erzeugten Druck im Reaktor von 200-550 mbar. Das freiwerdende Ammoniak wird zur weiteren Verwendung abgeführt, während nach beendeter Reaktion, welche durch das Ende der $NH_3$-Entwicklung gut kontrolliert werden kann, das im Sulfolan gelöste 1,2,4-Triazolidin-3,5-dion beim Abkühlen auskristallisiert und durch Absaugen, Abnutschen oder Zentrifugieren isoliert werden kann.

Die Kristallisation kann, falls gewünscht, durch Zugabe von inerten Lösungsmitteln, z.B. Toluol, beschleunigt werden, wobei die erhaltene Mutterlauge im nächsten Ansatz wiederverwendet werden kann und das vorhandene Toluol, speziell bei Verwendung von nutschenfeuchtem Hydrazodicarbonamid, als Schleppmittel für das azeotrop abzudestillierende Wasser dient. Das erhaltene kristalline 1,2,4-Triazolidin-3,5-dion kann normalerweise sofort, häufig sogar ohne vorherige Isolierung, weiterverarbeitet werden. Ein zusätzlicher Reinigungseffekt wird erzielt, wenn in die heiße 1,2,4-Triazolidin-3,5-dion enthaltende Sulfolan-Lösung Wasser zugegeben, wird, so daß das Triazolidin-3,5-dion aus dem Wasser/Sulfolan-Gemisch in sehr reiner Form auskristallisiert.

Wird die Cyclisierung in mit Wasser nicht oder wenig mischbaren Lösungsmitteln durchgeführt, wie solchen der Gruppe B und z.B. Diphenylether, so wird in der Regel nach der Cyclisierung zu dem abkühlenden Gemisch, gegebenenfalls unter Zuhilfenahme einer Rückkühlvorrichtung, bei Temperaturen ≤ 150 °C unter kräftigem Rühren Wasser zugetropft bzw. zugesetzt. Hierbei löst sich das 1,2,4-Triazolidin-3,5-dion in der wäßrigen Phase. Es wird so viel Wasser zugesetzt, daß 10 bis 60 gewichtsprozentige wäßrige Lösungen des 1,2,4-Triazolidin-3,5-dions erhalten werden. Die heiße wäßrige Lösung wird dann von der organischen Phase getrennt und durch Abkühlen das 1,2,4-Triazolidin-3,5-dion zum Kristallisieren gebracht. Nach dem Trocknen erhält man praktisch reines 1,2,4-Triazolidin-3,5-dion, welches in der Regel sofort weiterverarbeitet werden kann.

Selbstverständlich kann die Löslichkeit des 1,2,4-Triazolidin-3,5-dions in Wasser durch Zusatz von Lauge, z.B. Natronlauge führt zur Bildung des entsprechenden 1,2,4-Triazolidin-3,5-dion-Natriumsalzes, erhöht werden, so daß zum Entfernen des 1,2,4-Triazolidin-3,5-dions aus dem Reaktionsgefäß eine geringe Menge Wasser ausreicht, womit der Durchsatz pro Ansatz erhöht wird. Das entstandene Salz kann isoliert und für weitere Reaktionen weiterverwendet werden, es kann aber nach Zugabe von Säuren, z.B. Salzsäure, zum reinen 1,2,4-Triazolidin-3,5-dion umgesetzt werden, welches nach dem Kristallisieren durch Filtrieren, Abnutschen, Zentrifugieren isoliert werden kann.

Die beim erfindungsgemäßen Verfahren eingesetzten, mit Wasser nicht mischbaren organischen Lösungsmittel werden nach dem Abtrennen der wäßrigen 1,2,4-Triazolidin-3,5-dion-Lösung in der Regel für die nächsten Ansätze wiederverwendet, ebenso die wäßrigen Mutterlaugen für die Abtrennung und Isolierung des 1,2,4-Triazolidin-3,5-dions.

1,2,4-Triazolidin-3,5-dion ist ein wertvoller Ausgangsstoff zur Herstellung von temperaturbeständigen Bausteinen. Das hieraus hergestellte Trishydroxyalkyl-triazolidin-3,5-dion z.B. findet als vernetzende Komponente in temperaturbeständigen Elektroisolierlacken Verwendung, während das aus 1,2,4-Triazolidin-3,5-dion hergestellte Triglycidyl-triazolidin-3,5-dion z.B. als Vernetzer in Pulverlacken, die nach dem elektrostatischen Pulversprühverfahren angewendet werden, eingesetzt wird. Ferner wird 1,2,4-Triazolidin-3,5-dion in photographischen Zusammensetzungen eingesetzt.

Die in den Beispielen angegebenen Prozente beziehen sich auf das Gewicht.

Beispiel 1

In einem 6-l-Dreihalskolben, der mit Rührer, Thermometer, Tropftrichter und Destillationsbrücke ausgestattet ist, werden 3 kg Sulfolan und 1,18 kg an der Luft getrocknetes Hydrazodicarbonamid innerhalb 2,5 Stunden auf 200 °C aufgeheizt, wobei zu Beginn der Ammoniakentwicklung bei 150-160 °C leichtes Vakuum angelegt wird. Dann wird die Temperatur innerhalb einer Stunde auf 210 °C erhöht. Nach ca. 1,5 Stunden entstand eine klare Lösung, die noch ca. 3,5 Stunden bei 210 °C und 200 mbar bis zur Beendigung der Reaktion weitergerührt wurde. Das restliche Ammoniak wird nach Abkühlen auf 180 °C bei 40 bis 80 mbar entfernt. Zu der abkühlenden Lösung werden bei Normaldruck 0,8 kg Toluol so zugetropft, daß praktisch kein Toluol abdestilliert. Das auskristallisierte, fast reine 1,2,4-Triazolidin-3,5-dion wird nach dem Abkühlen auf Raumtemperatur abgesaugt und mit Toluol gewaschen. Es werden 0,87 kg (= 86,2 % d.Th.) getrocknetes 1,2,4-Triazolin-3,5-dion mit einer Reinheit von 97,5 %, bestimmt durch Titration mit n/10 Natronlauge gegen Phenolphthalein, erhalten.

Beispiel 2-4

Die in Beispiel 1 erhaltene Mutterlauge wird, wie in Beispiel 1 beschrieben, zum Cyclisieren von 1,18 kg Hydrazodicarbonamid verwendet. Das in der Mutterlauge gelöste Toluol destilliert beim Aufheizen ab und kann zum Kristallisieren wiederverwendet werden. Die Ausbeute beträgt in allen 3 Ansätzen zwischen 94 und 97 % d.Th. bei gleichbleibender Reinheit des erhaltenen 1,2,4-Triazolidin-3,5-dions.

Beispiel 5

In einer 1 l-Rührapparatur analog Beispiel 1 werden 400 g N-Methyl-pyrrolidon und 160 g nutschenfeuchtes Hydrazodicarbonamid (enthält 73,8 % trockenes Hydrazodicarbonamid) auf 205 °C aufgeheizt. Das ab 160 °C freiwerdende Ammoniak wird durch Ausblasen mit Stickstoff entfernt. Nach 3,5-stündiger Reaktion bei 205 °C werden im Wasserstrahlvakuum ca. 300 g Lösungsmittel abdestilliert und die verbleibende Lösung mit Aceton versetzt. Das auskristallisierte Triazolidin-3,5-dion wird nach dem Abkühlen auf Raumtemperatur abgesaugt, mit Aceton gewaschen und getrocknet. Es werden 74 g eines 97,5 %igen 1,2,4-Triazolidin-3,5-dions erhalten.

Aus der Mutterlauge können noch 16 g eines 96,5 %igen 1,2,4-Triazolidin-3,5-dions isoliert werden, so daß die Gesamtausbeute 90 g (= 89 % d.Th.) beträgt.

Beispiel 6

Beispiel 6 soll zeigen, daß die Reinheit des nach dem erfindungsgemäßen Verfahren hergestellten rohen 1,2,4-Triazolidin-3,5-dions für eine weitere Umsetzung ohne Isolierung ausreichend ist.

In einem 500 ml-Vierhalskolben, der mit Rührer, Thermometer und Rückflußkühler versehen ist, werden 59 g Hydrazodicarbonamid in 100 g Sulfolan analog Beispiel 1 cyclisiert. Beim Abkühlen kristallisiert das 1,2,4-Triazolidin-3,5-dion so konzentriert aus, daß zwecks besserer Rührbarkeit noch 45 g Sulfolan zugesetzt werden. Nach Zugabe von 0,5 g Tetraethylammoniumchlorid werden in die erhaltene Suspension bei 120 °C im Verlauf von 6 Stunden durch ein nachträglich installiertes Gaseinleitungsrohr 66 g Ethylenoxid so eingeleitet, daß kein Ethylenoxid entweicht. Nach beendeter Reaktion wird das Lösungsmittel im Vakuum bei 0,3 mbar entfernt. Es werden 120 g rohes N,N',N"-Tris-(2-hydroxyethyl)-triazolidin-3,5-dion erhalten, welches laut gaschromatographischer Analyse 92 % reines Triol und 2,8 % Sulfolan enthält. Dieses rohe Tris-(2-hydroxyethyl)-triazolidin-3,5-dion, welches nach einigen Stunden bei Raumtemperatur zu kristallisieren beginnt, kann ungereinigt oder nach vorherigem Umkristallisieren aus z.B. 3 Teilen Isopropanol und 7 Teilen Aceton gereinigt, zur Herstellung von z.B. temperaturbeständigen Polymeren, z.B. Polyestern oder Polyesterimiden, z.B. zum Isolieren von Kupferdrähten verwendet werden.

Beispiel 7

In einem 2 l-Dreihalskolben, der mit Rührer, Thermometer und Rückflußkühler ausgestattet ist, werden, 600 g technisches m-Kresol und 236 g Hydrazodicarbonamid unter Überleiten von Stickstoff in 90 Minuten auf 195 °C aufgeheizt, 5,5 Stunden bei 195 °C gerührt und zu der abkühlenden Lösung 200 ml Toluol getropft. Die auf Raumtemperatur abgekühlte Suspension wird abgesaugt, mit Toluol gewaschen und getrocknet. Es werden 160 g eines 97 %igen 1,2,4-Triazolidin-3,5-dions erhalten.

Beispiel 8

In einem 2 l-Vierhalskolben, der mit Rührer, Thermometer, Tropftrichter und Destillationsbrücke ausgestattet ist, werden 600 g l-Methylnaphthalin und 300 g feuchtes Hydrazodicarbonamid (enthält 22,3 % Wasser) auf 200 °C erhitzt und 5 Stunden bei 200 °C und leichtem Vakuum gerührt. Während des Abkühlens werden 300 g Wasser zugetropft und die heiße wäßrige Phase abgetrennt, aus der beim Abkühlen ein praktisch reines 1,2,4-Triazolidin-3,5-dion auskristallisiert, welches durch Absaugen isoliert werden kann. Es werden 121 g getrocknetes 1,2,4-Triazolidin-3,5-dion mit einer Reinheit von 96,7 %, bestimmt durch Titration mit n/10 Natronlauge gegen Phenolphthalein, erhalten.

Beispiel 9

300 g feuchtes Hydrazodicarbonamid (enthält 22,3 % Wasser) werden in der organischen Phase aus Beispiel 8, wie im Beispiel 8 beschrieben, cyclisiert, Während des Abkühlens werden 150 g Wasser und die wäßrige Mutterlauge aus Beispiel 8 zugetropft. Nach dem Absaugen der kristallisierten Triazolidin-3,5-dion-Lösung werden 163 g getrocknetes 1,2,4-Triazolidin-3,5-dion mit einer Reinheit von 95,2 % erhalten.

Beispiel 10

In einem 6 l-Vierhalskolben, an dem ein durch ein Hähnchen zu öffnender Ablauf angebracht ist, und der mit Rührer, Thermometer, Tropftrichter und Destillationsbrücke ausgestattet ist, werden 1 800 g Diphenylmethan und 1 200 g feuchtes Hydrazodicarbonamid (enthält 22,3 % Wasser) auf 205 °C erhitzt, wobei zu Beginn der Ammoniakentwicklung bei 160-170 °C leichtes Vakuum angelegt wird. Es wird dann 5 Stunden bei 205 °C nachgerührt, wobei das Vakuum auf 200 mbar gesteigert wird. Zu der abkühlenden Lösung werden bei Normaldruck 2,4 kg Wasser so zugetropft, daß kein Wasser abdestilliert. Die heiße wäßrige Lösung wird ablaufen und unter Rühren abkühlen gelassen. Das auskristallisierte, fast reine 1,2,4-Triazolidin-3,5-dion wird abgesaugt und mit 400 g Wasser gewaschen. Es werden 528 g getrocknetes 1,2,4-Triazolidin-3,5-dion mit einer Reinheit von 97,9 % erhalten.

Beispiel 11-13

Das in Beispiel 10 erhaltene Diphenylmethan wird, wie in Beispiel 10 beschrieben, zum Cyclisieren von 1 200 g feuchtem Hydrazodicarbonamid (enthält 22,3 % Wasser) verwendet. Zum Abtrennen des erhaltenen rohen 1,2,4-Triazolidin-3,5-dions wird dieses in 2 600 g wäßriger Lösung, bestehend aus Mutterlauge und Waschwasser aus dem Beispiel 10, gelöst und analog Beispiel 10 aufgearbeitet. Die Ausbeute beträgt in allen 3 Ansätzen zwischen 730 und 760 g 1,2,4-Triazolidin-3,5-dion bei einer Reinheit zwischen 96,2 und 97,4 %.

Beispiel 14

236 g getrocknetes Hydrazodicarbonamid und 600 g Diphenylether werden in einem 2 l-Vierhalskolben, der mit Rührer, Thermometer, Tropftrichter und Rückflüßkühler versehen ist, auf 205 °C aufgeheizt, wobei zu Beginn der Ammoniakentwicklung bei 160 °C leichtes Vakuum angelegt wird, welches innerhalb 6 Stunden auf 300 mbar erhöht wird. Zu der abkühlenden Lösung werden bei Normaldruck 400 g Wasser zugetropft und die heiße wäßrige Lösung abgetrennt. Das auskristallisierte, fast reine 1,2,4-Triazolidin-3,5-dion wird nach dem Abkühlen auf Raumtemperatur abgesaugt und mit Wasser gewaschen. Es werden 132 g getrocknetes 1,2,4-Triazolidin-3,5-dion mit einer Reinheit von 98,3 % erhalten.

Beispiel 15

236 g getrocknetes Hydrazodicarbonamid und 600 g Diphenylether werden analog Beispiel 14 cyclisiert. Das rohe 1,2,4-Triazolidin-3,5-dion wird in der wäßrigen Mutterlauge aus Beispiel 14 gelöst, abgetrennt und zum Kristallisieren gebracht. Nach dem Absaugen werden 193 g getrocknetes 1,2,4-Triazolidin-3,5-dion mit einer Reinheit von 94,2 % erhalten.

**Ansprüche**

1. Verfahren zur Herstellung von 1,2,4-Triazolidin-3,5-dion aus Hydrazodicarbonamid durch Cyclisierung bei erhöhten Temperaturen unter Abspaltung von Ammoniak, dadurch gekennzeichnet, daß Hydrazodicarbonamid in mindestens einem organischen, gegebenenfalls mit Wasser mischbaren Lösungsmittel suspendiert und bei Temperaturen von 150 bis 280 °C und einem Druck von 50 mbar bis 5 bar und Entfernung des abgespaltenen Ammoniaks aus dem Reaktionsgemisch cyclisiert und das entstandene 1,2,4-Triazolidin-3,5-dion nach Kristallisation isoliert wird.

2. Verfahren gemäß Anspruch 1, dadurch gekennzeichnet, daß die Cyclisierung bei Temperaturen von 190-220 °C und bei einem Reaktionsdruck von 150 mbar bis atmosphärischem Druck durchgeführt wird.

3. Verfahren gemäß den Ansprüchen 1 und 2, dadurch gekennzeichnet, daß die Cyclisierung in mindestens einem organischen, polaren Lösungsmittel durchgeführt wird und nach der Cyclisierung mindestens ein weiteres, mit dem Cyclisierungs-Lösungsmittel mischbares Lösungsmittel oder Wasser dem Reaktionsgemisch zugesetzt wird.

4. Verfahren gemäß den Ansprüchen 1 und 2, dadurch gekennzeichnet, daß die Cyclisierung in mindestens einem organischen, mit Wasser nicht mischbaren Lösungsmittel durchgeführt wird und nach der Cyclisierung während des Abkühlens bei Temperaturen von etwa 60 °C bis 150 °C unter Rühren, gegebenenfalls unter Rückfluß, Wasser in einer solchen Menge zugesetzt wird, daß 10 bis 60 %ige wäßrige Lösungen des 1,2,4-Triazolidin-3,5-dions erhalten werden, aus denen das nach Abkühlen auf Zimmertemperatur auskristallisierende 1,2,4-Triazolidin-3,5-dion isoliert wird.

5. Verfahren gemäß Anspruch 3, dadurch gekennzeichnet, daß die nach dem Abtrennen des 1,2,4-Triazolidin-3,5-dions anfallende Mutterlauge wiederverwendet wird.

6. Verfahren gemäß Anspruch 4, dadurch gekennzeichnet, daß das nach dem Abtrennen der wäßrigen 1,2,4-Triazolidin-3,5-dion-Lösung zurückbleibende organische Lösungsmittel wiederverwendet wird.

7. Verfahren gemäß Anspruch 4, dadurch gekennzeichnet, daß die nach dem Abtrennen des kristallinen 1,2,4-Triazolidin-3,5-dions anfallende wäßrige Mutterlauge wiederverwendet wird.

## Claims

1. Process for the preparation of 1,2,4-triazolidine-3,5-dione from hydrazodicarbonamide by cyclisation at elevated temperatures with elimination of ammonia, characterised in that hydrazodicarbonamide is suspended in at least one organic, optionally water-miscible solvent and cyclised at temperatures of 150 to 280 °C and at a pressure of 50 mbar to 5 bar with removal of the ammonia split off from the reaction mixture, and the resulting 1,2,4-triazolidine-3,5-dione is isolated after crystallisation.

2. Process according to Claim 1, characterised in that cyclisation is carried out at temperatures of 190 to 220 °C and at a reaction pressure of 150 mbar to atmospheric pressure.

3. Process according to Claims 1 and 2, characterised in that cyclisation is carried out in at least one organic polar solvent, and at least one other solvent, which is miscible with the cyclisation solvent, or water is added to the reaction mixture after cyclisation.

4. Process according to Claims 1 and 2, characterised in that cyclisation is carried out in at least one organic solvent which is immiscible with water, and after cyclisation, while the reaction mixture is cooling down, at temperatures of about 60 °C to 150 °C, water is added with stirring, optionally under reflux, in such a quantity that 10 to 60 % aqueous solutions of 1,2,4-triazolidine-3,5-dione are obtained, from which the 1,2,4-triazolidine-3,5-dione which crystallises after cooling to room temperature is isolated.

5. Process according to Claim 3, characterised in that the mother liquor obtained after removal of the 1,2,4-triazolidine-3,5-dione is reused.

6. Process according to Claim 4, characterised in that the organic solvent remaining after removal of the aqueous 1,2,4-triazolidine-3,5-dione solution is reused.

7. Process according to Claim 4, characterised in that the aqueous mother liquor obtained after the separation of crystalline 1,2,4-triazolidine-3,5-dione is reused.

## Revendications

1. Procédé de préparation de la 1,2,4-triazolidine-3,5-dione à partir de l'hydrazodicarboxamide par cyclisation à température élevée avec séparation d'ammoniac, caractérisé en ce que l'on met en suspension l'hydrazodicarboxamide dans au moins un solvant organique éventuellement miscible à l'eau et on cyclise à des températures de 150 à 280 °C sous une pression de 50 mbars à 5 bars en éliminant l'ammoniac libéré du mélange de réaction, et on isole la 1,2,4-triazolidine-3,5-dione formée après cristallisation.

2. Procédé selon la revendication 1, caractérisé en ce que l'on cyclise à des températures de 190 à 220 °C et sous une pression de réaction allant de 150 mbars jusqu'à la pression atmosphérique.

3. Procédé selon les revendications 1 et 2, caractérisé en ce que l'on cyclise dans au moins un solvant organique polaire et, après cyclisation, on ajoute au mélange de réaction au moins un autre solvant miscible avec le solvant de cyclisation ou de l'eau.

4. Procédé selon les revendications 1 et 2, caractérisé en ce que l'on cyclise dans au moins un solvant organique non miscible à l'eau et, après la cyclisation, au cours du refroidissement à des températures d'environ 60 à 150 °C, sous agitation, éventuellement au reflux, on ajoute de l'eau en quantité suffisante pour obtenir des solutions aqueuses de 1,2,4-triazolidine-3,5-dione à une concentration de 10 à 60 % à partir desquelles, après refroidissement à température ambiante, on isole la 1,2,4-triazolidine-3,5-dione qui cristallise.

5. Procédé selon la revendication 3, caractérisé en ce qu'on réutilise les liqueurs mères obtenues après séparation de la 1,2,4-triazol-idine-3,5-dione.

6. Procédé selon la revendication 4, caractérisé en ce qu'on réutilise le solvant organique restant après séparation de la solution aqueuse de 1,2,4-triazolidine-3,5-dione.

7. Procédé selon la revendication 4, caractérisé en ce qu'on réutilise les liqueurs mères aqueuses obtenues après séparation de la 1,2,4-triazolidine-3,5-dione cristallisée.